# EUROPEAN PATENT SPECIFICATION

(11) **EP 3 077 064 B1**
(45) Date of publication and mention of the grant of the patent: **07.06.2023**
(21) Application number: 14867681.0
(22) Date of filing: 24.10.2014
(51) Int. Cl.: A62B 18/08, A62B 18/02, A62B 17/04, A61M 16/10

(54) **AN ACTIVE VENTING SYSTEM AND DEVICES INCORPORATING ACTIVE VENTING SYSTEM**
AKTIVES ENTLÜFTUNGSSYSTEM UND VORRICHTUNGEN MIT DEM AKTIVEN ENTLÜFTUNGSSYSTEM
SYSTÈME DE VENTILATION ACTIVE ET DISPOSITIFS CONTENANT UN SYSTÈME DE VENTILATION ACTIVE

(30) Priority: 04.12.2013 US 201361911995 P; 31.12.2013 SG 2013097191
(43) Date of publication of application: 12.10.2016
(73) Proprietor: ST ENGINEERING INNOSPARKS PTE. LTD., Singapore 139953 (SG)
(72) Inventor: TANG, Ee Ho, Singapore 259283 (SG); SAK, Kwok Jiang, Singapore 542297 (SG); LEE, Wei Liang Jerome, Singapore 786879 (SG)
(74) Representative: Dummett Copp LLP
(86) International application number: PCT/SG2014/000498
(87) International publication number: WO 2015/084255

(56) References cited:
- EP-A1- 0 558 147
- WO-A1-90/05565
- WO-A2-2014/035641
- CN-A- 1 189 384
- CN-A- 103 404 982
- GB-A- 2 226 490
- US-A- 4 549 542
- US-A- 4 646 732
- US-A- 4 646 732
- US-A- 5 104 430
- US-A- 5 318 020
- US-A1- 2005 103 343
- US-A1- 2006 076 012
- US-A1- 2007 240 716
- US-A1- 2010 083 967
- US-A1- 2013 139 816
- US-B1- 6 257 235

## Description

### TECHNICAL FIELD

The present disclosure relates generally to an active venting system (AVS) for use in protective gears and respiratory devices such as masks, helmets, covering nose and face of wearer. More particularly, present invention refers to a respiratory device with an active venting system to effectively remove exhaled air in every breathing cycle.

### BACKGROUND

Protective gears and respiratory devices such as masks, helmets, etc., that covers nose and face of a wearer need venting systems to remove warm and humid exhaled air inside them during its operation. The venting system can be with or without forced ventilation.

US 20110083255 discloses a venting system for a sports helmet with air venting openings wherein at least some of the venting openings have covers that at least partially cover the venting openings. By tilting about an axis of rotation, the covers partially or completely release the venting openings. Upon actuation of the cover, one end of the cover is lifted above the surrounding helmet surface while the other end of the cover is lowered below the level of the surrounding helmet surface. Thus, the air inside the sports helmet is vented by actuation of the cover by the user and not by the forced ventilation.

US2013/0139816 A1 discloses a portable personal respiratory protection device including a self-contained head gear for agricultural use. The respirator includes a power source, a cooling source such as a fan, an air filter and a Bluetooth wireless communication for communicating with other mask users.

WO90/05565 A1 discloses a protective hood for use in fire emergencies or as protection against irritant gases. The hood comprises a head cover with filter pads to cover the nose and mouth and a fan to permit the removal of accumulated air along with a power source to drive the fan motor. The power is supplied to the fan motor automatically as soon as the hood is first donned.

US2010/0083967 A1 discloses a mask device with a blower and a sensor for warning and detecting the movement position of an exhaust valve in controlling the internal pressure of the mask device.

US2005/0103343 A1 discloses a respiratory system for a gas mask including a pressure and control device to control the air flow from a blower. During exhalation by a user the pressure or state of the outflow valve is detected in the mask and the air flow is accordingly altered.

US 4646732 discloses a respiratory mask with a casing connected to fix a driving motor, a supplying air fan with canisters to supply air to the mask via the fans and an exhausting air fan for sucking air from the mask.

US 6257235 B1 discloses a face mask having a filter body sized to cover the nose and mouth of a wearer, a securing mechanism for holding the filter body in close fit with the wearer's face, and a fan attachment to enhance breathability and comfort of the mask. The fan is configured directly against said exterior surface of said filter body so that when operated said fan draws at least a portion of its air flow directly through said filter body thereby communicating with said interior air volume through said filter body.

N95 masks and respirators are used as protective gears against air pollutants such as haze and influenza. Commercially available products without forced ventilation are classified into two main categories: single-use disposable respirators and reusable respirators with detachable filter cartridges. These products without forced ventilation often come with a one-way integrated valve (passive valves) to allow the exhaled air to escape. However, the efficacy of these passive valves in removing the warm and humid exhaled air is highly dependent on breathing depth and pressure.

Often, the exhaled air is forced to exit through the filters due to the limited window of the one-way valve. Moisture in the warm exhaled air condenses as it flows through the cold filters and blocks the air channels within the filters partially or completely. The reduction in the size of the air channels directly increases air resistance which causes breathing difficulties. In addition, lack of a proper exhalation pathway causes the hot and humid exhaled air to circulate within the respirator body thus, creating discomfort to the user.

To counter this problem, respirators with forced ventilation or air supply powered by fans and/or blowers are available. These devices are designed to supply significant airflow of up to 800L/min to cater to the breathing needs of an individual. These products are typically bulky and require significant power supply to maintain a prolong airflow and pressure. The power supply is usually external and not part of the respirator body to meet the high airflow rates. These products are therefore, not suitable for daily usage of general public.

Therefore, an active venting system is needed which when incorporated into respiratory device or protective gears, can be used comfortably for prolong usage and is suitable for general public. Further, it is required that such device should minimize facial contact of the hot and humid exhaled air which causes discomfort to a wearer and have an effective means of exhausting heat and humidity in each exhalation cycle.

### SUMMARY

According to the invention there is provided a respiratory device comprising:
a respirator body which covers face and mouth of a wearer to form a relatively closed area sealed from ambient air;
at least one first filter integrally disposed in the respirator body for filtering inhaled air; and
at least one active venting system functionally affixed to the internal surface of the respirator body wherein the active venting system operates to vent air from the respirator body, wherein the active venting system comprises a housing comprising:
   a power source;
   a blower coupled to the power source; and
   an electronic device or a printed circuit board coupled to the blower,
      the blower is a micro-fan and is turned on or off by the electronic device or printed circuit board to vent exhaled air from the respirator body into the atmosphere,
   the blower allows active air exchange and to vent air into the atmosphere from within the respirator body,
   the electronic device or the printed circuit board triggers control signals for the blower, and
   an air guide surrounding the nose and mouth region of the wearer to channel and separate exhaled air and inhaled air,
      characterized in that
   the air guide is circular in shape and is attached to the inner side of the respirator body,
   the active venting system is positioned within the circumference of the air guide,
   the air guide is positioned at an angle inclined with respect to an ergonomic facial profile; and the air guide is positioned so that at least one gap exists between the air guide and the base of the respirator body to allow excess exhaled air to escape in case the excess exhaled air is not vented through the active venting system.

Preferred embodiments are defined in the dependent claims.

Other aspects and advantages of the invention will become apparent from the following detail description, taken in conjunction with the accompanying drawings, illustrating by way of example the principles of the invention.

### BRIEF DESCRIPTION OF THE DRAWINGS

The summary above, as well as the following detailed description of illustrative embodiments, is better understood when read in conjunction with the appended drawings. For the purpose of illustrating the present disclosure, exemplary constructions of the disclosure are shown in the drawings. However, the disclosure is not limited to specific methods and instrumentalities disclosed herein. Wherever possible, like elements have been indicated by identical numbers.
**FIGS. 1A-1B** are exemplary illustrations of a side view of a respiratory device;
**FIG. 2** is an exemplary illustration of a side view of the respiratory device of **FIG. 1** showing the direction of air flow during an exhalation phase;
**FIG. 3** is an exemplary illustration of a top perspective view of the respiratory device with the active venting system, air guide and filter;
**FIG. 4** is an exemplary illustration of a perspective view of the air guide within the respiratory device;
**FIG. 5** is an exemplary illustration of a perspective view of active venting system;
**FIG. 6** is an exemplary illustration of a top perspective view of the respiratory device showing establishment of a hot air zone during exhalation;
**FIG. 7** is an exemplary illustration of a top perspective view of the respiratory device showing establishment of a cool air zone during inhalation;
**FIG. 8** is an exemplary illustration of a top perspective view of the respiratory device showing an embodiment of the blower with a filter;
**FIG 9** is an exemplary illustration of a side view of another embodiment of the blower with a one way control valve; and
**FIG 10** is an exemplary illustration of a side view of another embodiment of the active venting system attached to a latch.

### DETAILED DESCRIPTION OF ILLUSTRATIVE EMBODIMENTS

The particular configurations discussed in these non-limiting examples can be varied and are cited merely to illustrate at least one embodiment and are not intended to limit the scope thereof.

Present invention relates to an active venting system comprising a power source; a blower coupled to the power source; and an electronic device or a printed circuit board coupled to the blower, wherein the blower is turned on or off by the electronic device or printed circuit board to vent the exhaled air from the respirator body.

When the respirator body is worn, the exhalation jet is blocked, and the air gets recirculated, resulting in increased heat, moisture and carbon dioxide within the respirator body. The active venting system of present invention solves this problem by mimicking the natural way of breathing. The active venting system has sufficient power to vent out the exhalation, up to 1m away. Thus the expired air is prevented from being recirculated within the respirator body.

The active venting system of instant invention not only removes the heat and moisture, but also removes the carbon dioxide. The active venting system can be adapted into all sort of protective gears such as helmets. The active venting system can also be adapted onto an escape hood. This is usually used for emergency situations, and covers the whole head or into any respiratory device. The respiratory device has a folding mechanism for easy storage and portability.

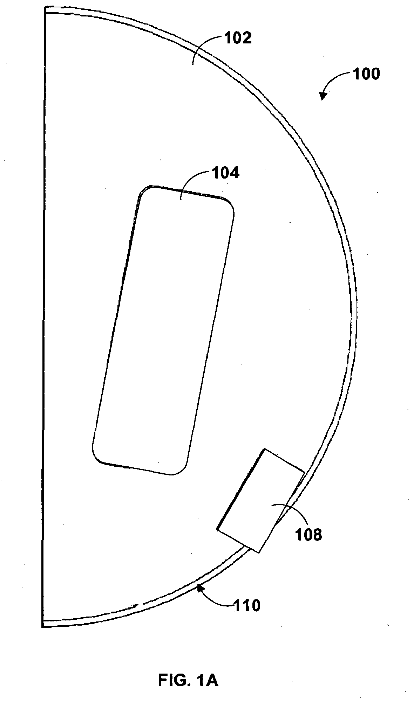

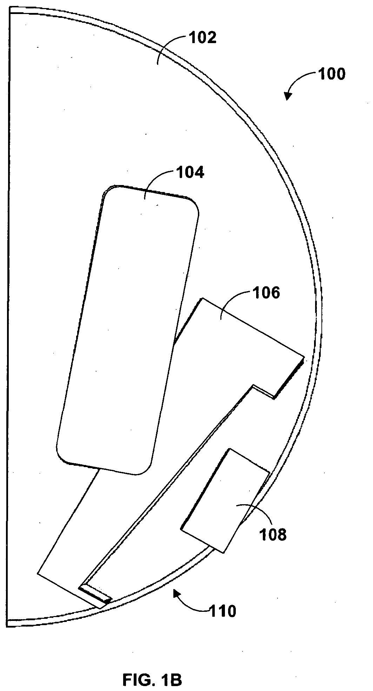

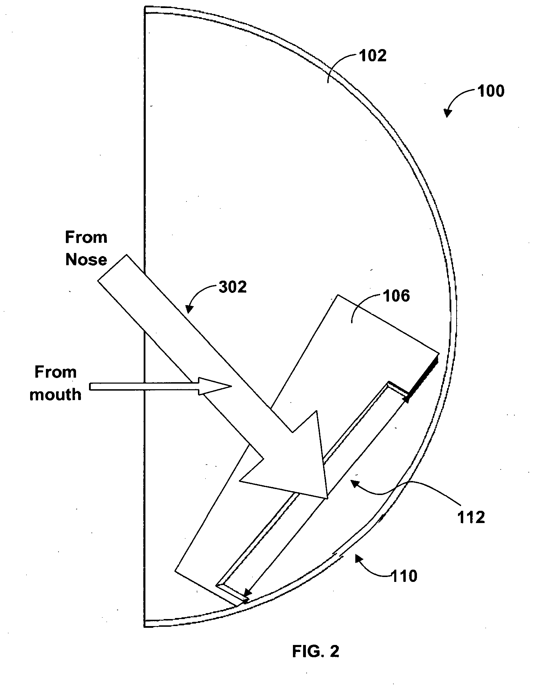

One embodiment of a respiratory device **100** depicted in **FIG. 1A** shows a side view of the respiratory device **100.** The respiratory device **100** comprises of a respirator body **102** with one or more filters **104** and an active venting system **108.** **FIG. 1B** illustrates a side view of a respiratory device **100** in accordance with another embodiment of the present invention, wherein the respiratory device **100** further comprises of an air guide **106.The** side views of the respiratory device **100** depicted in **FIGS. 1A-1B** show only one filter **104.** The filter **104** is integrally disposed on surface of the respirator body **102** so as to filter the inhaled air that passes through the respirator body **102.** The filter **104** can be, for example, disposable or replaceable air filters and made of without limitation, paper, foam or woven fiberglass. The filter **104** may have numerous perforations through which the air passes and is filtered before it enters the respirator body **102.** In the depicted embodiment, two filters **104,** a single air guide **106** and active venting system **108** are provided with the respirator body **102,** however, it is possible to apply the teachings of the invention with multiple such elements. It should be noted that the **FIGS. 2-10** are the illustrations of various views of **FIG. 1B****.**

As shown in **FIGS. 1B****,** **2** **and** **4****,** the air guide **106** is positioned near a base **110** of the respirator body **102** to channel the exhaled air from the nostrils and separate the exhaled air from the inhaled air around the nostrils region. According to the invention, the air guide **106** is circular in shape in the form of a ring and made of for example, silicone or a flexible polymer. The air guide **106** is attached to the inner side of the respirator body **102** via molding or heat joining.

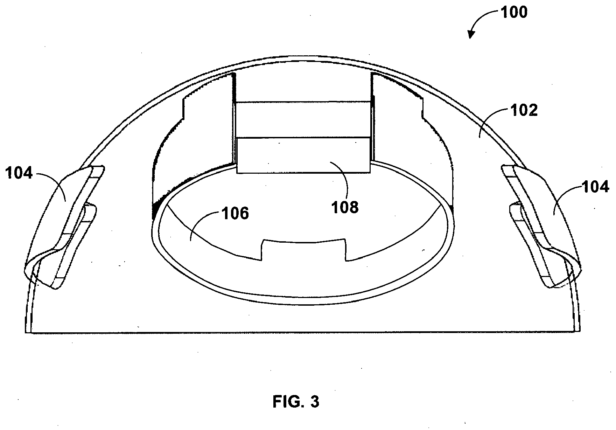

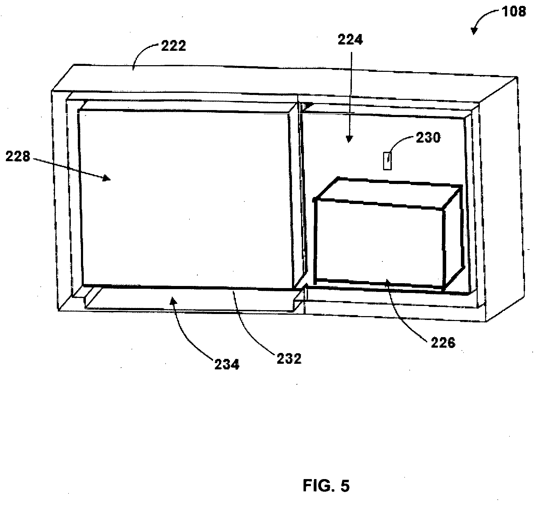

In the depicted embodiment as shown in **FIGS 1A-1B** and 3, the active venting system **108** is also positioned near the base **110** of the respirator body **102** such that the active venting system **108** is within the circumference of the air guide **106.** The active venting system **108** is affixed to the internal surface of the respirator body **102** and is used to remove the exhaled air from the respiratory device **100.** The positioning of the active venting system **108** along with the air guide **106** facilitates removal of the exhaled air during an exhalation cycle. The active venting system **108** may remain turned on throughout its operation, or it can be timed to operate only during the exhalation cycle. In the latter case, the active venting system **108** is activated by an embedded sensor. The speed and amount of air flow may be regulated by controlling a micro blower (explained in **FIG. 5**) provided in the active venting system **108** using suitable control signals. The filter **104,** air guide **106** and active venting system **108** are positioned to create separate air zones such that inhaled air flows through a region separate from the exhaled air and the inhaled air does not mix with the exhaled air.

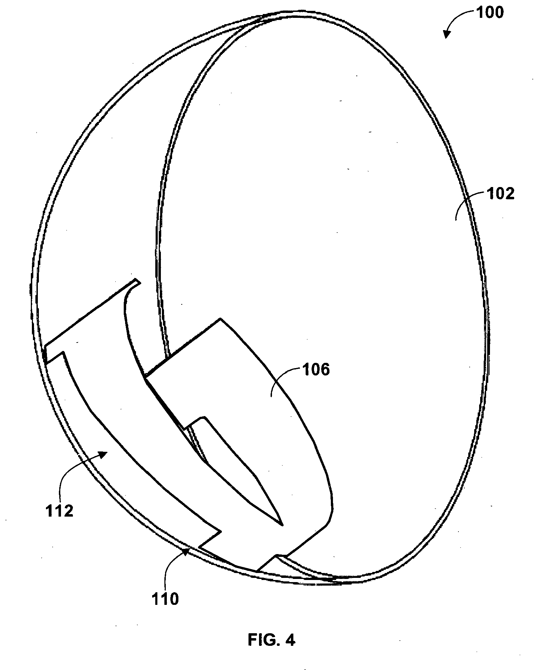

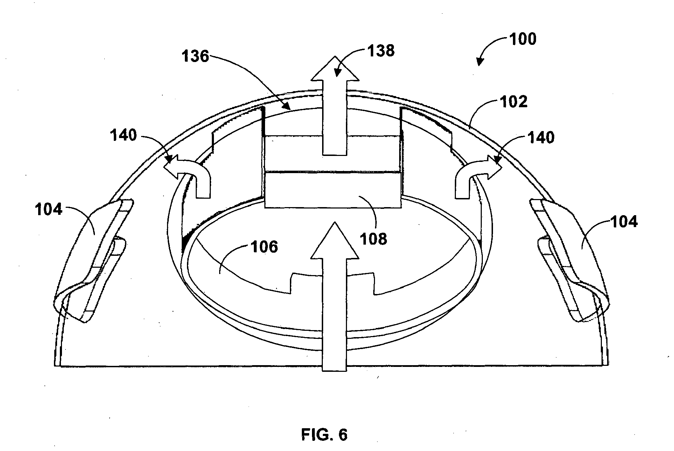

**FIG. 3** illustrates a top perspective view of the respiratory device **100.** The positioning of the filters **104,** air guide **106** and active venting system **108** is shown in the respirator body **102.** In an embodiment, the air guide **106** is positioned just below the nostrils such that the exhaled air falls directly in the space circumscribed by the air guide **106.** Due to this positioning, the air guide **106** channels the exhaled air into the active venting system **108** effectively (arrow **302** shown in **FIG 2**), and prevents the warm exhaled air to be directed to other parts of the respirator body **102.** Hence, the air guide **106** creates a segregation of a warm air zone along the exhalation stream from the rest of the respirator body **102** (further described in **FIG 6**). The exhaled air is then removed by the active venting system **108.** In addition, gaps **112** (shown in **FIGS. 2** and **4**) are provided between the air guide **106** and the respirator body **102** for the exhaled air to escape in case the speed of the active venting system **108** is not fast enough. The gaps **112** also allow some warm exhaled air to tentatively escape along the sides of the respirator body **102,** instead of reflecting back to the face after hitting the surface of the respirator body **102.**

Thus, during an inhalation cycle, the ambient air from outside the respirator body **102** flows through the air filters **104** into the relative closed area within the respirator body **102.** The internal air guide **106** and active venting system **108** effectively remove exhaled air during each exhaling cycle which is typically hot and humid.

Referring to **FIG. 4****,** a perspective view of the respiratory device **100** showing air guide **106** with gaps **112** is disclosed. The air guide **106** is positioned near the base **110** of the respirator body **102** such that one or more gaps **112** are provided between the respirator body **102** and the air guide **106.** The gaps **112** redirect air to other areas of the respirator body **102** during an exhalation cycle. The air guide **106** allows excess warm air to flow along the surface of the respirator body **102,** if it is not rapidly vented. The air guide **106** creates a warm air zone, segregated from the rest of the respirator body **102** and skin. It should be noted that the air guide **106** is not in contact with the face and may be angled within the respirator body to capture maximum exhaled air. This angled air guide **106** forms a channel for the exhaled air from a wearer's mouth and nose into the active venting system **108.**

One embodiment of the active venting system has been disclosed in **FIG. 5** of the invention. The active venting system **108** has a housing **222** which houses an electronic device or a printed circuit board **224,** a power source **226,** a blower **228,** and optionally, a sensor **230.** It is to be noted that other placements of the various components of the active venting system **108** are possible and are within the scope of the present invention. The electronic device **224** may be a processing unit that controls the functioning of various components of the active venting system **108.** Alternately, the electronic device **224** may include batteries and circuits only for embodiments where the blower **228** is turned on all the time. The electronic device **224** is coupled to the power source **226,** the blower **228,** and the sensor **230.** The blower **228** is operated by the power source **226** for example, a rechargeable battery. The power source **226** may be removed and charged at desired intervals. According to an embodiment of the invention, the active venting system may include a second filter (not shown).

The blower **228** is turned on or off by the electronic device or the printed circuit board **224** to vent the exhaled air. The electronic device or the printed circuit board **224** triggers suitable control signals for the blower **228** to regulate the speed and amount of airflow. According to an embodiment of the invention, the active venting system **108** remains turned on throughout the period of use. The active venting system **108** may be turned off manually after use.

According to an embodiment of the invention, the power source **226** is a detachable and chargeable power source.

In one embodiment the power source **226** is supplied by means of an electrical wire or cable to the electrical mains. In another embodiment the power source **226** recharged via a Universal Serial Bus (USB) micro cable to power bank whilst the active venting system is still operating.

In an embodiment, the blower **228** may be a micro blower or a micro fan providing for example, 0-25L/min of airflow. The blower **228** allows active air exchange within the respirator body **102** during an exhalation cycle. The sensor **230** may be for example, a piezoelectric sensor and embedded on the inner surface of the respirator body **102** in front of the nose or mouth to sense exhaled air during each breathing cycle. The sensor **230** in turn communicates with the electronic device **224** to control the operation of the blower **228.** Thereafter, the blower **228** effectively vents the respirator body **102** during each breathing cycle. In case sensor **230** is not present, the blower **228** is left on permanently.

It should be noted that the blower **228** in the active venting system **108** can be a range of different micro-fans, both of axial and centrifugal type. In one embodiment the blower **228** is an axial type micro-fan that sucks air from one direction and vents in another direction along the axis of the fan. Another embodiment of the present invention uses a centrifugal type micro-fan. One another embodiment use blower type centrifugal micro-fans. Both centrifugal micro-fan and blower type centrifugal micro-fans sucks air from one (or multiple) direction, and vents air through another direction(s).

In an embodiment, the blower **228** may create a forced ventilation to vent excess heat and humidity from the blower outlet **232** in each breathing cycle. Also, the opening **234** for blower egress may vent the exhaled air from the respirator body **102.** Further, the exhaled air can be filtered before exiting from the respirator body **102** through the optional filter. It should be noted that the blower **228** vents the air expelled by the user which is a carbon-di-oxide gas along with heat and moisture.

The invention substantially improves user's breathing comfort over long periods. The sensor **230** driven blower **228** is energy efficient and consumes significantly less power than conventional systems. In addition, the respiratory device **100** is lightweight, compact and portable due to the use of a micro blower/fan. The invention is an ideal solution for users who stay in a hostile environment with air pollution or contamination for a prolonged period of time.

**FIG. 6** depicts the functioning of the respiratory device **100** during an exhalation phase. Due to the angled positioning of the air guide **106,** the exhaled air concentrates in a region surrounded by the air guide **106.** The air guide **106** channels the exhaled air from the nostrils in this region and a hot air zone **136** is established inside the respirator body **102.** The sensor **230** depicted in **FIG. 5****,** senses the exhaled air due to which the electronic device **224** activates the blower **228.** The blower **228** then vents the exhaled air to the atmosphere as depicted by the arrow **138.**

It should be noted that most of the exhaled air is vented through the active venting system **108.** However, some of the exhaled air may escape from the gaps **112** as depicted by arrows **140.** The gaps **112** allow excess exhaled air to escape to the sides of the respirator body **102** when the speed of the active venting system **108** is not enough to vent the exhaled air completely.

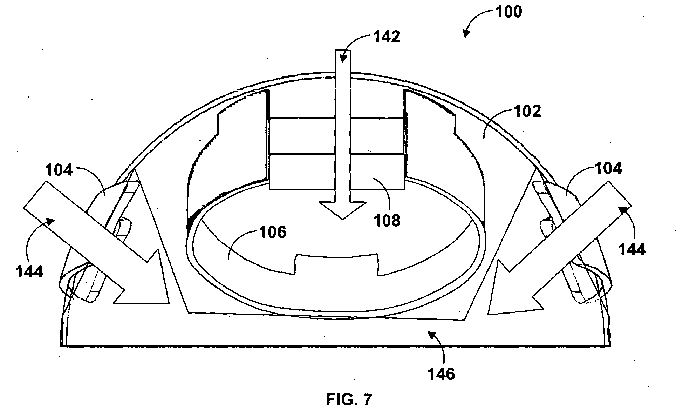

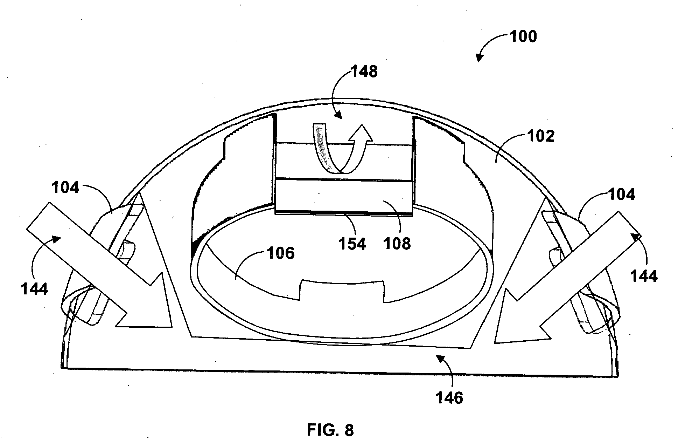

During inhalation, as shown in **FIG. 7****,** through the filter **104,** clean air enters the respirator body **102** depicted by airflow **144.** This creates a cool air zone **146** inside the respirator body **102.** Mostly, during inhalation, air does not enter through the blower outlet **232** as the blower **228** operates to vent out air, thereby preventing dust particles from entering through the blower outlet **232** anyway. However, in an optional embodiment, in order to prevent external dust particles from entering the respirator body **102** through the blower outlet **232** (depicted in **FIG. 5****)** as depicted by airflow **142,** a filter or a one-way valve may be used as described in **FIGS. 8-9****.**

**FIG. 8** depicts the embodiment wherein a second filter **154** is placed across a blower suction inlet. When air is being sucked through the blower outlet **232** in the reverse direction **148** during inhalation, particles are arrested by the second filter **154,** and prevented from entering the respirator body **102.** This embodiment has the additional advantage of minimizing humid air inside the respirator body **102** from being sucked into a blower motor and degrading the electronic device **224** during exhalation. It should be noted that the second filter **154** can be attached either to the blower inlet or outlet without limitation

In case the second filter **154** is not used, the blower expels air even during inhalation. Therefore, ambient air does not enter into the respirator body **102** as the blower continuously expels the air.

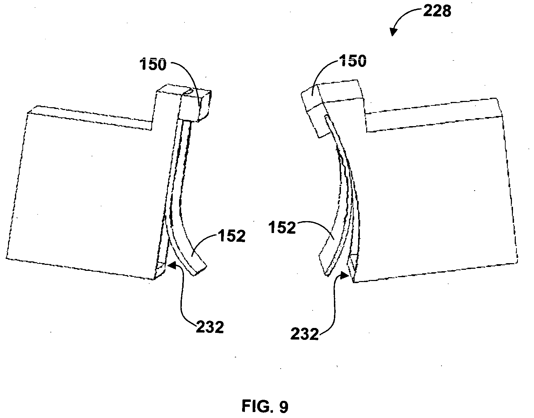

**FIG. 9** shows a second embodiment that implements one way control valve, using a membrane **152.** The membrane **152** allows air to flow out through the blower outlet **232,** but prevents air from entering in the reverse direction. The membrane **152** is attached to the blower outlet **232** by utilizing a fastener **150.** It is understood that a variety of mechanical means allow the membrane to be held over the blower outlet **232.** It should be noted that the one way control valve with a membrane can be attached either to the blower inlet or outlet without limitation.

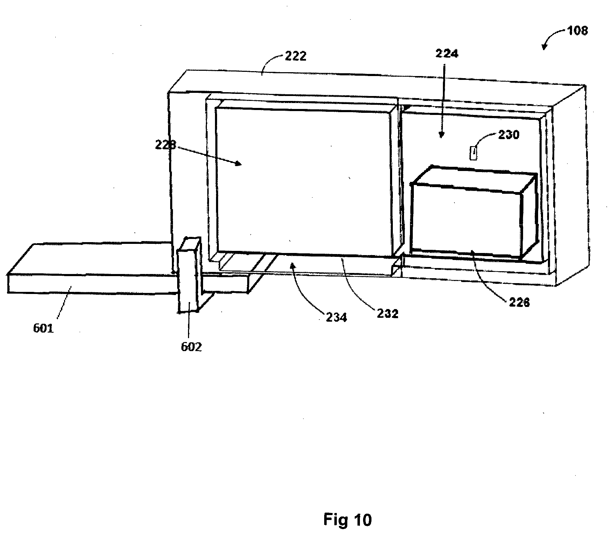

**Fig 10** shows another embodiment wherein the active venting system **108** is attached to a latch **601** with help of a latch holder **602** to prevent the ambient air from entering the respirator body **102.** The latch **601** is embedded with a sensor that automatically closes when the power of the active venting system **108** runs out. Also if required the user can manually operate the latch **601** to close to block the blower opening. It should be noted that the latch **601** can be attached either to the blower inlet or outlet without limitation.

From the above, it should be noted that the air guide **106,** positioned at the bottom of the respirator body **102,** along with the active venting system **108** creates a dedicated pathway for exhaled air to be effectively vented from the respirator body **102.** In fact, dedicated pathways are formed due to the described positioning of the filters **104,** air guide **106** and active venting system **108.** Inhaled air flows through a region separate from the exhaled air and does not mix with the exhaled air forming separate air zones.

Further, the active venting system **108** can be adapted and attached onto any respirators or protective gears. The respirator body may be used, for example, a disposable N95 respirator. In the respirator body of N95 respirator, an opening can be cut and the active venting system **108** can be attached to it. Similarly, the active venting system **108** can be fit onto the respirator body **102** of other respirators (disposable or non-disposable alike). The disposable respirators can be for example the silicon/rubber ones, full-face or half-face, with filters or canisters, etc without limitation.

Through the above description, one can understand that this respiratory device with active venting system and internal air guide can provide the wearer with filtered purified air, discharge exhaled air and vent the respirator body in every breathing cycle. All these features will provide users with an improved breathe experience due to the active air exchange within the respirator body.

## Claims

1. A respiratory device (100) comprising:
a respirator body (102) which covers face and mouth of a wearer to form a relatively closed area sealed from ambient air;
at least one first filter (104) integrally disposed in the respirator body (102) for filtering inhaled air; and
at least one active venting system (108) functionally affixed to the internal surface of the respirator body (102) wherein the active venting system (108) operates to vent air from the respirator body (102), wherein the active venting system comprises a housing (222) comprising:
a power source (226);
a blower (228) coupled to the power source (226); and
an electronic device or a printed circuit board (224) coupled to the blower (228),
the blower (228) is a micro-fan and is turned on or off by the electronic device or printed circuit board (224) to vent exhaled air from the respirator body (102) into the atmosphere,
the blower (228) allows active air exchange and to vent air into the atmosphere from within the respirator body (102),
the electronic device or the printed circuit board (224) triggers control signals for the blower (228), and
an air guide (106) surrounding the nose and mouth region of the wearer to channel and separate exhaled air and inhaled air,
**characterized in that**
the air guide (106) is circular in shape and is attached to the inner side of the respirator body (102),
the active venting system (108) is positioned within the circumference of the air guide (106),
the air guide (106) is positioned at an angle inclined with respect to an ergonomic facial profile; and the air guide (106) is positioned so that at least one gap (112) exists between the air guide (106) and the base of the respirator body (102) to allow excess exhaled air to escape in case the excess exhaled air is not vented through the active venting system (108).

2. The respiratory device (100) of claim 1, wherein the active venting system (108) is attached to a one way control valve with a membrane (152) to prevent ambient air from entering through the blower inlet or outlet into the respirator body (102) at all times; or the active venting system (108) is attached to a second filter (154) to prevent ambient air from entering the respirator body (102).

3. The respiratory device (100) of claim 2, wherein the one way control valve with the membrane (152) or the second filter (154) is attached either to an inlet or outlet of the blower.

4. The respiratory device (100) of any of the claims 1 to 3, wherein the inlet or the outlet of the blower is attached to a latch (601) to prevent ambient air from entering the respirator body (102).

5. The respiratory device (100) of claim 4, wherein the latch (601) is embedded with a sensor that automatically closes when the power of the active venting system (108) runs out; or the latch (601) is manually closed by user to block the blower opening.

6. The respiratory device (100) of claim 1, wherein the respirator body (102), the active venting system (108) and the air guide (106) are together collapsible into a pocket size.

7. The respiratory device (100) of any of the claims 1 to 6, wherein the respirator body (102) is any of the following: an air filtering respirator; an air purifying respirator; a disposable or reusable mask; a non-disposable mask; or an escape hood.

## Patentansprüche

1. Beatmungsgerät (100), umfassend:
einen Beatmungsmaskenkörper (102), der Gesicht und Mund eines Trägers bedeckt, um einen vergleichsweise geschlossenen Bereich, der von Umgebungsluft abgedichtet ist, zu bilden;
wenigstens einen ersten Filter (104), der integral in dem Beatmungsmaskenkörper (102) angeordnet ist, zum Filtern von eingeatmeter Luft; und
wenigstens ein aktives Entlüftungssystem (108), das funktionsfähig an der Innenoberfläche des Beatmungsmaskenkörpers (102) angebracht ist, wobei das aktive Entlüftungssystem (108) arbeitet, um Luft aus dem Beatmungsmaskenkörper (102) zu entlüften, wobei das aktive Entlüftungssystem eine Gehäuse (222) umfasst, umfassend:
eine Stromquelle (226);
ein Gebläse (228), das an die Stromquelle (226) gekoppelt ist; und
eine elektronische Vorrichtung oder eine Leiterplatte (224), die an das Gebläse (228) gekoppelt ist,
wobei das Gebläse (228) ein Mikrolüfter ist und von der elektronischen Vorrichtung oder Leiterplatte (224) ein- oder ausgeschaltet wird, um ausgeatmete Luft aus dem Beatmungsmaskenkörper (102) an die Atmosphäre zu entlüften,
wobei das Gebläse (228) aktiven Luftaustausch erlaubt und Entlüften von Luft von innerhalb des Beatmungsmaskenkörpers (102) an die Atmosphäre erlaubt,
wobei die elektronische Vorrichtung oder die Leiterplatte (224) Steuersignale für das Gebläse (228) auslöst und
eine Luftführung (106), die die Nase und den Mundbereich des Trägers umgibt, um ausgeatmete Luft und eingeatmete Luft zu kanalisieren und zu trennen,
**dadurch gekennzeichnet, dass**
die Luftführung (106) kreisförmig geformt ist und an der Innenseite des Beatmungsmaskenkörpers (102) angebracht ist,
das aktive Entlüftungssystem (108) innerhalb des Umfangs der Luftführung (106) angeordnet ist,
die Luftführung (106) unter einem schrägen Winkel bezogen auf ein ergonomisches Gesichtsprofil angeordnet ist; und die Luftführung (106) so angeordnet ist, dass wenigstens eine Lücke (112) zwischen der Luftführung (106) und der Basis des Beatmungsmaskenkörpers (102) vorliegt, um zu erlauben, dass überschüssige ausgeatmete Luft entweicht, wenn die überschüssige ausgeatmete Luft nicht durch das aktive Entlüftungssystem (108) entlüftet wird.

2. Beatmungsgerät (100) gemäß Anspruch 1, wobei das aktive Entlüftungssystem (108) an einem Einweg-Steuerventil mit einer Membran (152) angebracht ist, um zu allen Zeiten zu verhindern, dass Umgebungsluft durch den Gebläseeinlass oder -auslass in den Beatmungsmaskenkörper (102) eintritt; oder das aktive Entlüftungssystem (108) an einem zweiten Filter (154) angebracht ist, um zu verhindern, dass Umgebungsluft in den Beatmungsmaskenkörper (102) eintritt.

3. Beatmungsgerät (100) gemäß Anspruch 2, wobei das Einweg-Steuerventil mit einer Membran (152) oder der zweite Filter (154) entweder an dem Einlass oder dem Auslass des Gebläses angebracht ist.

4. Beatmungsgerät (100) gemäß einem der Ansprüche 1 bis 3, wobei der Einlass oder der Auslass des Gebläses an einer Verriegelung (601) angebracht ist, um zu verhindern, dass Umgebungsluft in den Beatmungsmaskenkörper (102) eintritt.

5. Beatmungsgerät (100) gemäß Anspruch 4, wobei in die Verriegelung (601) eine Sensor eingebettet ist, der automatisch schließt, wenn der Strom des aktiven Entlüftungsgeräts (108) zu Ende geht; oder die Verriegelung (610) von einem Benutzer von Hand geschlossen wird, um die Gebläseöffnung zu blockieren.

6. Beatmungsgerät (100) gemäß Anspruch 1, wobei der Beatmungsmaskenkörper (102), das aktive Entlüftungssystem (108) und die Luftführung (106) gemeinsam auf Taschengröße zusammenlegbar sind.

7. Beatmungsgerät (100) gemäß einem der Ansprüche 1 bis 6, wobei der Beatmungsmaskenkörper (102) eines der folgenden ist: ein Luftfilter-Atemgerät; ein Luftreinigungs-Atemgerät; eine Einweg- oder Mehrwegmaske; eine Nicht-Einwegmaske; oder eine Brandfluchthaube.

## Revendications

1. Dispositif respiratoire (100) comprenant:
un corps de respirateur (102) qui couvre le visage et la bouche d'un porteur pour former une zone relativement fermée rendue étanche à l'air ambiant;
au moins un premier filtre (104) disposé intégralement dans le corps de respirateur (102) pour filtrer l'air inhalé, et
au moins un système d'évacuation active (108) fixé fonctionnellement à la surface interne du corps de respirateur (102) dans lequel le système d'évacuation active (108) fonctionne pour évacuer l'air du corps de respirateur (102), dans lequel le système d'évacuation active comprend un boîtier (222) comprenant:
une source d'alimentation (226);
une soufflerie (228) couplée à la source d'alimentation (226); et
un dispositif électronique ou une carte de circuit imprimé (224) couplé à la soufflerie (228),
la soufflerie (228) est un micro-ventilateur et est activée ou désactivée par le dispositif électronique ou la carte de circuit imprimé (224) pour évacuer l'air expiré du corps de respirateur (102) dans l'atmosphère,
la soufflerie (228) permet un échange d'air actif et d'évacuer l'air dans l'atmosphère depuis l'intérieur du corps de respirateur (102),
le dispositif électronique ou la carte de circuit imprimé (224) déclenche des signaux de commande pour la soufflerie (228), et
un guide d'air (106) entourant la région du nez et de la bouche du porteur pour canaliser et séparer l'air expiré et l'air inhalé,
**caractérisé en ce que**
le guide d'air (106) est de forme circulaire et est fixé au côté intérieur du corps de respirateur (102),
le système d'évacuation active (108) est positionné à l'intérieur de la circonférence du guide d'air (106),
le guide d'air (106) est positionné selon un angle incliné par rapport à un profil facial ergonomique; et le guide d'air (106) est positionné de sorte qu'au moins un espace (112) existe entre le guide d'air (106) et la base du corps de respirateur (102) pour permettre à l'air expiré en excès de s'échapper au cas où l'air expiré en excès n'est pas évacué par l'intermédiaire du système d'évacuation active (108).

2. Dispositif respiratoire (100) selon la revendication 1, dans lequel le système d'évacuation active (108) est fixé à une soupape de commande unidirectionnelle avec une membrane (152) pour empêcher l'air ambiant d'entrer par l'entrée ou la sortie de la soufflerie dans le corps de respirateur (102) à tout moment; ou le système d'évacuation active (108) est fixé à un deuxième filtre (154) pour empêcher l'air ambiant d'entrer dans le corps de respirateur (102).

3. Dispositif respiratoire (100) selon la revendication 2, dans lequel la soupape de commande unidirectionnelle avec la membrane (152) ou le second filtre (154) est fixée à une entrée ou à une sortie de la soufflerie.

4. Dispositif respiratoire (100) selon l'une quelconque des revendications 1 à 3, dans lequel l'entrée ou la sortie de la soufflerie est fixée à un loquet (601) pour empêcher l'air ambiant d'entrer dans le corps de respirateur (102).

5. Dispositif respiratoire (100) selon la revendication 4, dans lequel le loquet (601) est intégré avec un capteur qui se ferme automatiquement lorsque l'alimentation du système d'évacuation active (108) est épuisée; ou le loquet (601) est fermé manuellement par l'utilisateur pour bloquer l'ouverture de la soufflerie.

6. Dispositif respiratoire (100) selon la revendication 1, dans lequel le corps de respirateur (102), le système d'évacuation active (108) et le guide d'air (106) sont pliables ensemble en un format de poche.

7. Dispositif respiratoire (100) selon l'une quelconque des revendications 1 à 6, dans lequel le corps de respirateur (102) est l'un quelconque des suivants: un respirateur filtrant l'air; un respirateur purifiant l'air; un masque jetable ou réutilisable; un masque non jetable; ou une hotte d'évacuation.
